# EUROPÄISCHE PATENTANMELDUNG

(11) **EP 3 838 391 A1**
(43) Veröffentlichungstag der Anmeldung: **23.06.2021**
(21) Anmeldenummer: 19217310.2
(22) Anmeldetag: 18.12.2019
(51) Int. Cl.: B01F 15/02, B01F 13/00, A61B 17/88

(54) **VORRICHTUNG ZUM BEREITSTELLEN VON KNOCHENZEMENT**

(71) Anmelder: Heraeus Medical GmbH, 61273 Wehrheim (DE)
(72) Erfinder: ROKITTA, Hermann Josef, 45481 Mülheim an der Ruhr (DE)
(74) Vertreter: Heraeus IP

(57) **Zusammenfassung**

Die Erfindung betrifft eine Vorrichtung 100 zum Bereitstellen eines Knochenzements aus zwei Ausgangskomponenten, umfassend ein zylinderartiges erstes Behältnis 200 mit einem ersten Innenraum 220, in dem ein Knochenzementpulver 225 als erste Ausgangskomponente lagerbar ist, ein zylinderartiges zweites Behältnis 300 mit einem zweiten Innenraum 320, in dem ein Kolben-Zylinder-System 600 angeordnet ist, aufweisend einen in einem Zylinder 620 angeordneten Kolben 610, wobei Kolben und Zylinder relativ zueinander axial bewegbar sind und das Kolben-Zylinder-System ein Fördervolumen 630 aufweist, ein erstes Leitungsmittel 670 welches das Fördervolumen fluidleitend mit dem ersten Innenraum verbindet, ein Lagerelement 400 mit einem dritten Innenraum 420, in dem eine Monomerflüssigkeit 425 als zweite Ausgangskomponente lagerbar ist, wobei das erste Behältnis 200 und das zweite Behältnis 400 axial miteinander verbunden sind. Die Erfindung betrifft weiterhin ein Verfahren zum Bereitstellen eines Knochenzements mit der erfindungsgemäßen Vorrichtung.

## Beschreibung

### Beschreibung

Die Erfindung betrifft eine Vorrichtung zum Bereitstellen eines Knochenzements aus zwei Ausgangskomponenten, umfassend ein zylinderartiges erstes Behältnis mit einem ersten Innenraum, in dem ein Knochenzementpulver als erste Ausgangskomponente lagerbar ist, ein zylinderartiges zweites Behältnis mit einem zweiten Innenraum, in dem ein Kolben-Zylinder-System angeordnet ist, aufweisend einen in dem Zylinder angeordneten Kolben, wobei Kolben und Zylinder relativ zueinander axial bewegbar sind und das Kolben-Zylinder-System ein Fördervolumen aufweist, ein erstes Leitungsmittel welches das Fördervolumen fluidleitend mit dem ersten Innenraum verbindet, ein Lagerelement mit einem dritten Innenraum, in dem eine Monomerflüssigkeit als zweite Ausgangskomponente lagerbar ist, wobei das erste Behältnis und das zweite Behältnis axial miteinander verbunden sind. Die Erfindung betrifft weiterhin ein Verfahren zum Bereitstellen eines Knochenzements mit der erfindungsgemäßen Vorrichtung.

### Hintergrund der Erfindung

Es werden erhebliche Bemühungen unternommen, Vorrichtungen und Verfahren zum Bereitstellen von Knochenzement aufzuzeigen, mittels derer Knochenzement einfach, sicher und schnell bereitgestellt werden kann. Ein wichtiger Aspekt bei der Bereitstellung von Knochenzement ist die Vermeidung von Lufteinschlüssen im Knochenzement. Zu deren Vermeidung wurde eine Vielzahl von Vakuum-Zementiersystemen beschreiben, von denen exemplarisch folgende genannt sind: US 6,033,105 A, US 5,624,184 A, US 4,671,263 A, US 4,973,168 A, US 5,100,241 A, WO 99/67015 A1, EP 1020167 A2, US 5,586,821 A, EP 1016452 A2, DE 3640279 A1, WO 94/26403 A1, EP 1005901 A2, EP 1886647 A1, US 5,344,232 A.

Es besteht im Markt der Wunsch zur Vereinfachung der Bereitstellung von Knochenzement. Eine Vereinfachung besteht in der Bereitstellung von Zementiersystemen, in denen beide Ausgangskomponenten in separaten Bereichen der Systeme gelagert sind und erst unmittelbar vor der Zementierapplikation im Zementiersystem miteinander vermischt werden. Solche geschlossenen, sogenannten Full-Prepacked-Systeme, sind in folgenden Schriften genannt: EP 0692229 A1, DE 10 2009 031 178 B3, DE 10 2015 106 899 B3, US 5,997,544 A, US 6,709,149 B1, DE 698 12 726 T2, EP 0796653 A2, US 5,588,745 A.

In der DE 10 2016 121 607 A1 wird ein Full-Prepack-System beschrieben, wobei der mit Monomerflüssigkeit gefüllte Behälter axial hinter dem Knochenzement gelagert ist. Nachteilig ist, dass der mit Monomerflüssigkeit gefüllte Behälter im Zuge des Mischens der Ausgangkomponenten vollständig zerstört werden muss. Als ebenso nachteilig hat sich herausgestellt, dass der Krafteintrag zum Mischen der Ausgangskomponenten als auch später beim Austragen des gemischten Knochenzements immer auch auf die Überreste des zerstörten Behälters einwirkt. Dies führt zu einem erhöhten Kraftaufwand beim Anwender als auch zu ruckartigen und unkontrollierbaren Bewegungen sowohl im Zuge der Zerstörung der Behälter, als auch im Zuge des Mischens und späteren Austragens des gemischten Zements. Beides erschwert in erheblichem Maße die Anwendbarkeit des Full-Prepack-Systems, insbesondere im Zuge von zeitkritischen Operationsbedingungen. Als ebenso nachteilig hat sich herausgestellt, dass der zerstörte Behälter die Verwendung des Full-Prepack-Systems erschwert. Dies geschieht beispielsweise durch Bruchstücke des Behälters, die Monomerflüssigkeit zurückhalten, welche dann nicht zur Herstellung des Knochenzements zur Verfügung steht. Ein weiterer Nachteil entsteht durch Verkeilungen der Überreste des Behälters innerhalb der Misch - und Austragsvorrichtung. Ein weiterer Nachteil ist das Gefährdungspotential der Bruchstücke sowohl für den Anwender, als auch für den Patienten.

### Aufgaben

Eine Aufgabe der vorliegenden Erfindung ist es, einen oder mehrere der sich aus dem Stand der Technik ergebenden Nachteile zumindest teilweise zu überwinden.

Im Speziellen basiert die Erfindung auf dem Ziel, Vorrichtungen zum Bereitstellen eines Knochenzements aus zwei Ausgangskomponenten mit gleichbleibender Qualität bereitzustellen, welche zudem zuverlässig und sicher in der Anwendbarkeit sind. Das Ziel ist zudem, die Infektionsgefahr für den Patienten zu minimieren. Die Vorrichtungen sollen geeignet sein, die zwei Ausgangskomponenten separat voneinander lagern zu können. Die zwei Ausgangskomponenten sollen in der Vorrichtung innerhalb weniger Sekunden in der geschlossenen Vorrichtung zusammengeführt werden können. Die Vorrichtung soll den Knochenzement ohne eine mechanische Durchmischung der Ausgangskomponenten bereitstellen. Die Vorrichtung soll ohne extern angelegtes Vakuum in der Lage sein, den Knochenzement bereitzustellen. Die Vorrichtung soll ohne Umbaumaßnahmen und ohne externe Gerätschaften, wie beispielsweise Schläuche oder Vakuumquellen, den Knochenzement bereitstellen können. Die Vorrichtung soll mit möglichst wenigen Arbeitsschritte bedient werden können, um Fehlerquellen durch den Anwender zu minimieren.

Es ist eine weitere Aufgabe der Erfindung, ein Verfahren bereitzustellen, mit dem Knochenzement aus zwei Ausgangskomponenten bereitgestellt werden kann, mittels derer mindestens ein Teil der bereits beschriebenen Aufgaben zumindest zum Teil gelöst ist.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.
|1| Vorrichtung zum Bereitstellen eines Knochenzements aus zwei Ausgangskomponenten, umfassend
   ein zylinderartiges erstes Behältnis mit einem ersten Innenraum, in dem ein Knochenzementpulver als erste Ausgangskomponente lagerbar ist,
   ein zylinderartiges zweites Behältnis mit einem zweiten Innenraum, in dem ein Kolben-Zylinder-System angeordnet ist, aufweisend einen in einem Zylinder angeordneten Kolben, wobei Kolben und Zylinder relativ zueinander axial bewegbar sind und das Kolben-Zylinder-System ein Fördervolumen aufweist,
   ein erstes Leitungsmittel welches das Fördervolumen fluidleitend mit dem ersten Innenraum verbindet,
   ein Lagerelement mit einem dritten Innenraum, in dem eine Monomerflüssigkeit als zweite Ausgangskomponente lagerbar ist,
   wobei das erste Behältnis und das zweite Behältnis axial miteinander verbunden sind, und
   **dadurch gekennzeichnet, dass**
   das Lagerelement an einer Außenfläche des zweiten Behältnisses angeordnet ist,
   ein fluidleitendes zweites Leitungsmittel das Fördervolumen und den dritten Innenraum verbindendet,
   das Kolben-Zylinder-System
   - in einer ersten Position eine fluidleitende Verbindung zwischen dem zweiten Leitungsmittel und dem Fördervolumen eröffnet und
   - in einer zweiten Position die fluidleitende Verbindung zwischen dem zweiten Leitungsmittel und dem Fördervolumen verschließt, und
   die Vorrichtung ein Antriebselement umfasst, wobei das Antriebselement über eine Getriebeeinheit auf das Kolben-Zylinder-System einwirkt.
|2| Vorrichtung nach Ausführungsform 1, **dadurch gekennzeichnet,** dass die Getriebeeinheit ein zahnstangenartig ausgebildetes erstes Vortriebsmittel und ein wenigstens partiell ritzelartig ausgestaltetes zweites Vortriebsmittel umfasst.
|3| Vorrichtung nach einer der vorhergehenden Ausführungsformen 1 oder 2, **dadurch gekennzeichnet,** dass das Antriebselement derart mit dem Zylinder verbunden ist, dass eine rotatorische Bewegung des Antriebselements in einer linearen Bewegung des Zylinders resultiert.
|4| Vorrichtung nach einer der vorhergehenden Ausführungsformen 1 bis 3, **dadurch gekennzeichnet,** dass das Antriebselement hebelartig ausgestaltet und an der Außenfläche des zweiten Behältnisses angeordnet ist, insbesondere, dass das Antriebselement um eine Achse drehbar gelagert ist, bevorzugt um wenigstens 90 Grad, insbesondere wenigstens 60 Grad drehbar gelagert ist, insbesondere dass die Achse im Wesentlichen rechtwinklig zu einer Längsachse der Vorrichtung angeordnet ist.
|5| Vorrichtung nach einer der vorhergehenden Ausführungsformen 1 bis 4, **dadurch gekennzeichnet,** dass der Zylinder axial in dem zweiten Innenraum bewegbar ist.
|6| Vorrichtung nach einer der vorhergehenden Ausführungsformen 1 bis 5, **dadurch gekennzeichnet,** dass der Kolben form- und/oder kraftschlüssig mit dem zweiten Behältnis verbunden ist.
|7| Vorrichtung nach einer der vorhergehenden Ausführungsformen 4 bis 6, **dadurch gekennzeichnet,** dass das Antriebselement derart mit dem Zylinder verbunden ist, dass eine rotatorische Bewegung des Antriebselements um die Achse in einer linearen Bewegung des Zylinders resultiert.
|8| Vorrichtung nach einer der vorhergehenden Ausführungsformen 1 bis 4, **dadurch gekennzeichnet,** dass der Kolben axial in dem zweiten Innenraum bewegbar ist.
|9| Vorrichtung nach einer der vorhergehenden Ausführungsformen 1 bis 4 oder 8, **dadurch gekennzeichnet,** dass der Zylinder form- und/oder kraftschlüssig mit dem zweiten Behältnis verbunden ist.
|10| Vorrichtung nach einer der vorhergehenden Ausführungsformen 4, oder 8, **dadurch gekennzeichnet,** dass das Antriebselement derart mit dem Kolben verbunden ist, dass eine rotatorische Bewegung des Antriebselements um die Achse in einer linearen Bewegung des Kolbens resultiert.
|11| Vorrichtung nach einer der vorhergehenden Ausführungsformen, **dadurch gekennzeichnet,** dass die Vorrichtung, insbesondere das erste Behältnis, mischeinrichtungsfrei ist.
|12| Vorrichtung nach einer der vorhergehenden Ausführungsformen, **dadurch gekennzeichnet,** dass das Lagerelement ein Gefäß mit der Monomerflüssigkeit beinhaltet.
|13| Vorrichtung nach Ausführungsform 12, **dadurch gekennzeichnet,** dass das Lagerelement eine Öffnungsvorrichtung für das Gefäß aufweist, insbesondere dass die Öffnungsvorrichtung ein Anstechdorn oder eine Bruchkante ist.
|14| Vorrichtung nach Ausführungsform 12 oder 13, **dadurch gekennzeichnet,** dass das Gefäß eine Glasampulle oder ein Beutel ist.
|15| Vorrichtung nach einer der vorhergehenden Ausführungsformen, **dadurch gekennzeichnet,** dass in dem ersten Behältnis ein axial verschiebbarer Austragskolben angeordnet ist.
|16| Vorrichtung nach einer der vorhergehenden Ausführungsformen, **dadurch gekennzeichnet,** dass das Knochenzementpulver in dem ersten Behältnis zwischen dem Austragskolben und einer Austragsvorrichtung angeordnet ist.
|17| Vorrichtung nach einer der vorhergehenden Ausführungsformen, **dadurch gekennzeichnet,** dass das erste Behältnis ein erstes Anschlussmittel und das zweite Behältnis ein zweites Anschlussmittel aufweisen, um das erste Behältnis und das zweite Behältnis zu verbinden, insbesondere form- und/oder kraftschlüssig zu verbinden.
|18| Vorrichtung nach der vorhergehenden Ausführungsform 17, **dadurch gekennzeichnet,** dass das erste Anschlussmittel und das zweite Anschlussmittel einen Bajonettverschluss bilden.
|19| Vorrichtung nach der vorhergehenden Ausführungsform 18, **dadurch gekennzeichnet,** dass das erste Anschlussmittel als ein Außengewinde und das zweite Anschlussmittel als ein Innengewinde ausgestaltet sind, wobei das Außengewinde und das Innengewinde form- und/oder kraftschlüssig miteinander verbindbar sind.
|20| Verfahren zur Bereitstellung eines Knochenzements aus zwei Ausgangkomponenten mittels einer Vorrichtung gemäß einer der vorhergehenden Ausführungsformen, zumindest umfassend die folgenden sequenziellen Teilschritte:
   a) Überführen des Kolben-Zylinder-Systems in die erste Position,
   b) Fördern der Monomerflüssigkeit mittels der fluidleitenden Verbindung in das Fördervolumen,
   c) Überführen des Kolben-Zylinder-Systems aus der ersten Position in die zweite Position,
   d) Verschließen der fluidleitenden Verbindung zwischen dem zweiten Leitungsmittel und dem Fördervolumen,
   e) Fördern der Monomerflüssigkeit in den ersten Innenraum,
   f) Ausbildung des Knochenzements aus Knochenzementpulver und Monomerflüssigkeit.
|21| Verfahren nach Ausführungsform 20, **dadurch gekennzeichnet,** dass die Ausbildung des Knochenzements aus Knochenzementpulver und Monomerflüssigkeit ohne mechanische Einwirkung erfolgt.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A, bedeuten entsprechend als Werte Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht" oder "Parallelität" nie exakt, sondern nur innerhalb gewisser fertigungstechnischer Fehlertoleranzen gegeben sein kann. Beispielsweise schließen "im Wesentlichen parallele Achsen" einen Winkel von 85 Grad bis 95 Grad zueinander ein und "im Wesentlichen gleiche Volumen" umfassen eine Abweichung von bis zu 5 Volumen-%.

### Ausführliche Beschreibung

Ein erster Gegenstand der Erfindung betrifft eine Vorrichtung zum Bereitstellen eines Knochenzements aus zwei Ausgangskomponenten, umfassend ein zylinderartiges erstes Behältnis mit einem ersten Innenraum, in dem ein Knochenzementpulver als erste Ausgangskomponente lagerbar ist, ein zylinderartiges zweites Behältnis mit einem zweiten Innenraum, in dem ein Kolben-Zylinder-System angeordnet ist, aufweisend einen in einem Zylinder angeordneten Kolben, wobei Kolben und Zylinder relativ zueinander axial bewegbar sind und das Kolben-Zylinder-System ein Fördervolumen aufweist, ein erstes Leitungsmittel welches das Fördervolumen fluidleitend mit dem ersten Innenraum verbindet, ein Lagerelement mit einem dritten Innenraum, in dem eine Monomerflüssigkeit als zweite Ausgangskomponente lagerbar ist, wobei das erste Behältnis und das zweite Behältnis axial miteinander verbunden sind, und dadurch gekennzeichnet, dass das Lagerelement an einer Außenfläche des zweiten Behältnisses angeordnet ist, ein fluidleitendes zweites Leitungsmittel den zweiten Innenraum und den dritten Innenraum verbindendet, das Kolben-Zylinder-System in einer ersten Position eine fluidleitende Verbindung zwischen dem zweiten Leitungsmittel und dem Fördervolumen eröffnet und in einer zweiten Position die fluidleitende Verbindung zwischen dem zweiten Leitungsmittel und dem Fördervolumen verschließt, und die Vorrichtung ein Antriebselement umfasst, wobei das Antriebselement über eine Getriebeeinheit auf das Kolben-Zylinder-System einwirkt.

Die erfindungsgemäße Vorrichtung weist zylinderartige Behältnisse auf. Unter einem zylinderartigen Behältnis ist ein rohrartiges Gebilde zu verstehen, welches einen Innenraum und eine den Innenraum umgebende Behältniswand umfasst. Das zylinderartige Behältnis besitzt senkrecht zu einer Längsachse einen Querschnitt. Erfindungsgemäß kann es vorgesehen sein, dass ein Behältnis mehr als einen Innenraum umfasst. Beispielsweise kann ein Behältnis zwei Innenräume, drei Innenräume oder vier Innenräume umfassen. Umfasst ein Behältnis mehr als einen Innenraum, so sind diese Innenräume bevorzugt durch eine Trennwand oder ein Fördermittel voneinander abgetrennt. Erfindungsgemäß kann der Querschnitt der Behältnisse beliebige Formen annehmen. Beispielsweise kann der Querschnitt oval, quadratisch, fünfeckig, sechseckig, unregelmäßig oder kreisförmig ausgestaltet sein.

Es ist bevorzugt, dass das zylinderförmige Behältnis eine zylindrische Geometrie mit rotationssymmetrischer Achse mit rundem Querschnitt aufweist. Diese Geometrie erlaubt eine gute Handhabbarkeit für den Anwender und reduziert durch die Abwesenheit von Kanten ein Risiko einer Verkeilung beweglicher Teile innerhalb der Vorrichtung. Erfindungsgemäß können die Behältnisse und/oder die gesamte Vorrichtung unterschiedlichste Materialien oder Materialkombinationen aufweisen. Beispiele kann die Vorrichtung Kunststoff aufweisen oder aus Kunststoff bestehen. Bevorzugt handelt es sich bei dem Kunststoff um einen transparenten Kunststoff, da der Anwender auf diese Weise eine ordnungsgemäße Funktion der Vorrichtung während einer Verwendung der Vorrichtung optisch kontrollieren kann.
Eine Ausgestaltungsform der Vorrichtung ist dadurch gekennzeichnet, dass das erste zylinderförmige Behältnis und das zweite zylinderförmige Behältnis axial miteinander verbunden sind. Eine Verbindung ist axial, falls die beiden Längsachsen der hohlzylinderförmigen Behältnisse im Wesentlichen in die gleiche Richtung verlaufen und sich bevorzugt im Wesentlichen überlagern. Erfindungsgemäß sind bei axialer Verbundenheit die Querschnittsebenen der verbundenen Behältnisse im Wesentlichen parallel zueinander.

Die erfindungsgemäße Vorrichtung umfasst ein Lagerelement. Ein Lagerelement dient der Lagerung der Monomerflüssigkeit. Unter einem Lagerelement ist ein rohrartiges Gebilde zu verstehen, welches einen Innenraum und eine den Innenraum umgebende Wand umfasst. Die Monomerflüssigkeit kann dabei vorzugsweise steril und hermetisch dicht gelagert werden. Erfindungsgemäß kann das Lagerelement unterschiedlichste Materialien oder Materialkombinationen aufweisen. Beispiele kann das Lagerelement Kunststoff aufweisen oder aus Kunststoff bestehen.

In einer Ausgestaltungsform wird die Monomerflüssigkeit derart in dem Innenraum des Lagerelements gelagert, dass die Wand des Lagerelements direkt in Kontakt mit der Monomerflüssigkeit in Kontakt kommt. In einer weiteren, bevorzugten Ausgestaltungsform wird die Monomerflüssigkeit innerhalb des Lagerelements in einem separaten Gefäß gelagert.

Erfindungsgemäß werden unter einem separaten Gefäß alle Lagermöglichkeiten verstanden, welche die Monomerflüssigkeit hermetisch dicht und steril lagern können und durch manuelle Krafteinwirkung zerstörbar sind. Beispiele für Gefäße sind Glasampullen, Kunststoffampullen und Kunststoffbeutel. Aufgrund der leichten Sterilisierbarkeit und guten Öffenbarkeit durch manuelle Krafteinwirkung sind Glasampullen bevorzugt.

Die Anordnung des Lagerelements an einer Außenfläche des zweiten Behältnisses, bevorzugt an einer nicht auf einer Längsachse des zweiten Behältnisses befindlichen Außenfläche des zweiten Behältnisses, erlaubt ein Fördern der Monomerflüssigkeit durch das erste Leitungsmittel mittels des Kolben-Zylinder-Systems in das erste Behältnis, ohne dass das Kolben-Zylinder-System eine Krafteinwirkung auf das Lagerelement ausübt. Somit erfolgt auch kein Krafteintrag auf die vorzugsweise verwendete Glasampulle der Monomerflüssigkeit, wodurch nach einem Öffnen der Glasampulle und Ausfließen der Monomerflüssigkeit, die Glasampulle nicht vollständig zerstört werden muss. Die Folge ist ein geringerer Kraftaufwand für den Anwender und zugleich ein kontrolliertes Fördern der Monomerflüssigkeit in den ersten Innenraum, da keine Bruchstücke der Glasampulle innerhalb des Kolben-Zylinder-System vorhanden sind. Da die Glasampulle nur punktuell, aber nicht vollständig zerstört werden muss, besteht ein geringeres Risiko einer Verkeilung durch Bruchstücke der Glasampulle während der Verwendung der Vorrichtung. Ebenso ist das Verletzungsrisiko durch Bruchstücke sowohl für den Patienten, als auch für den Anwender, gesenkt.

Die Vorrichtung weist ein erstes Leitungsmittel und ein zweites Leitungsmittel auf. Erfindungsgemäß werden unter Leitungsmitteln alle Elemente verstanden, welche zwei Räume fluidleitend miteinander verbinden. Beispiele für solche Elemente sind rohrartige oder schlauchartige Verbindungen. Die Leitungsmittel sind bevorzugt derart ausgestaltet, dass zwar ein Flüssigkeitsaustausch stattfinden kann, allerdings ein Austausch von Feststoffen, wie beispielsweise von Knochenzementpulver oder Glasscherben einer Glasampulle, unterbunden wird. Dazu kann das Leitungsmittel beispielsweise ein Sieb oder eine Porenscheibe aufweisen. Das Sieb oder die Porenscheibe sind dabei derart gestaltet, dass ein schneller und vollständiger Austausch von Monomerflüssigkeit stattfinden kann und gleichzeitig ein Austausch von Feststoffen im Wesentlichen verhindert wird.

Um auf der einen Seite einen Austausch von größeren Feststoffen, wie beispielsweise Glasscherben zu unterbinden, kann das Leitungsmittel in Form einer hohlrohrartigen Verbindung beispielsweise einen Innenquerschnitt von kleiner/gleich 2 mm² aufweisen, bevorzugt von kleiner/gleich 1,5 mm². Um auf der anderen Seite einen möglichst schnellen und vollständigen Austausch von Monomerflüssigkeit zu ermöglichen kann das Leitungsmittel in Form einer hohlrohrartigen Verbindung beispielsweise einen Innenquerschnitt von mindestens 0,5 µm² aufweisen, bevorzugt von mindestens 1,0 mm². Beispielsweise kann das Leitungsmittel in Form einer hohlrohrartigen Verbindung einen Innenquerschnitt in einem Bereich von 0,5 bis 2 mm², bevorzugt von 1 bis 1,5 mm², aufweisen. In einer weiteren Ausgestaltungsform kann das Leitungsmittel ein Sieb aufweisen oder aus einem Sieb bestehen.

Das Kolben-Zylinder-System dient dem Fördern der Monomerflüssigkeit aus dem Fördervolumen über das erste Leitungsmittel in den ersten Innenraum. Das Kolben-Zylinder-System umfasst einen hohlen Zylinder mit einem Innenvolumen und einen im Zylinder axial beweglichen Kolben, wobei das Innenvolumen und der Kolben ein im Wesentlichen gleiches Volumen einnehmen. Durch die räumliche Lage von Kolben und Zylinder zueinander wird ein Fördervolumen ausgebildet, welches von einer Seite durch den Kolben und von den anderen Seiten durch den Zylinder umgrenzt ist. Kolben und Zylinder wirken derart miteinander, dass eine reversible Relativbewegung von Kolben und Zylinder aufeinander zu das Fördervolumen verkleinert, und, bei Anwesenheit einer Monomerflüssigkeit innerhalb des Fördervolumens, einen Transfer der Monomerflüssigkeit durch das erste Leitungsmittel in den ersten Innenraum zur Folge hat. Je nach räumlicher Lage des Kolbens, des Zylinders und des ersten Behältnisses zueinander, weist dabei entweder der Kolben oder der Zylinder das erste Leitungsmittel auf. Beispielsweise kann der Kolben das erste Leitungsmittel umfassen, falls der Kolben angrenzend an das erste Behältnis angeordnet ist.

Die Monomerflüssigkeit gelangt aus dem dritten Innenraum durch das zweite Leitungsmittel in das Fördervolumen. Um den Transfer zu erlauben, ist das Fördervolumen in der ersten Position des Kolben-Zylinder-Systems fluidleitend durch das zweite Leitungsmittel mit dem dritten Innenraum verbunden. Um die Monomerflüssigkeit anschließend durch das erste Leitungsmittel aus dem Fördervolumen in den ersten Innenraum zu fördern, muss das zweite Leitungsmittel in der zweiten Position des Kolben-Zylinder-Systems für die Monomerflüssigkeit verschlossen sein. In einer ersten Ausgestaltungsform verschließt eine Bewegung des Kolbens aus einer ersten Position in eine zweite Position das zweite Leitungsmittel. In einer weiteren, bevorzugten, Ausgestaltungsform verschließt eine Bewegung des Zylinders aus einer ersten Position in eine zweite Position das zweite Leitungsmittel. Die zweite Position des Kolben-Zylinder-Systems muss dabei erreicht werden, bevor der Transfer der Monomerflüssigkeit aus dem Fördervolumen in den ersten Innenraum einsetzt. Ansonsten würde ein Rücktransfer der Monomerflüssigkeit aus dem Fördervolumen in den dritten Innenraum erfolgen. Unter einer Position des Kolben-Zylinder-Systems ist eine räumliche Anordnung des Kolbens und des Zylinders des Kolben-Zylinder-Systems zueinander zu verstehen.

Kommt das Knochenzementpulver mit der Monomerflüssigkeit in Kontakt, hat dies ein sofortiges Anquellen des Knochenzementpulver zur Folge. Dies kann unter anderem zu einem Verstopfen des Leitungsmittel durch angequollenes Knochenzement führen, was ein vollständiges Fördern der Monomerflüssigkeit in den ersten Innenraum erschwert oder sogar verhindert. Eine unvollständige Vermischung der Ausgangskomponenten zieht sowohl Nachteile bei der Applizierfähigkeit des Knochenzements, als auch bei den mechanischen Eigenschaften des gehärteten Knochenzement, welche mit dem Mischungsverhältnis von Knochenzementpulver und Monomerflüssigkeit variieren, nach sich. Um das vollständige Volumen der Monomerflüssigkeit mit dem Knochenzementpulver in Kontakt zu bringen, sollte das Fördern durch das Kolben-Zylinder-System schnell und gleichmäßig erfolgen. Um dies zu gewährleisten, umfasst die Vorrichtung ein Antriebselement, welches mittels einer Getriebeeinheit das Kolben-Zylinder-System antreibt. Die Getriebeeinheit dient der Übersetzung einer vom Anwender initiierten Bewegung in eine axiale Relativbewegung von Kolben und Zylinder des Kolben-Zylinder-Systems zueinander. Die vom Anwender initiierte Bewegung kann dabei beispielsweise durch die Betätigung eines Hebels oder Griffs, der jeweils Teil des Antriebselements ist, ausgelöst werden. Die Verwendung eines Getriebes ist von Vorteil, da hierdurch die Möglichkeit gegeben ist, das Hebelprinzip auszunutzen, was zu einem zuverlässigen und gleichmäßigen Fördern der Monomerflüssigkeit führt. Zudem kann die Monomerflüssigkeit, falls notwendig, unter erhöhtem Förderdruck schnell, und somit vollständig, aus dem Fördervolumen in den ersten Innenraum gefördert werden. Somit kann der gewünschte Knochenzement anwendungssicher mit immer gleichbleibendem Mischungsverhältnis und damit in gleichbleibender Qualität zur Verfügung gestellt werden.

Unterstützt wird das Fördern der Monomerflüssigkeit zudem durch die axiale Verbindung des ersten Behältnisses zum zweiten Behältnis. Dadurch kann das Fördern der Monomerflüssigkeit auf einem direkten, das heißt geradlinigen, und möglichst kurzen Förderweg ohne Förderdruckverlust durch Biegungen und Kurven des ersten Leitungsmittel erfolgen. Ein geradliniges und kurzes erstes Leitungsmittel unterstützt somit ein schnelles und vollständiges Fördern der Monomerflüssigkeit. Eine axiale Verbindung zwischen erstem Behältnis und zweitem Behältnis verringert zudem den Platzbedarf und ermöglicht durch die kompakte Bauweise eine einfache und sichere Handhabbarkeit der Vorrichtung, was gerade im Zuge von zeitkritischen und oftmals hektischen Operationsbedingungen notwendig ist.

Die Getriebeeinheit kann unterschiedlich ausgestaltet sein, um die Kraftübertragung vom Antriebselement auf das Kolben-Zylinder-System zu bewerkstelligen. In einer Ausgestaltungsform weist die Getriebeeinheit ein erstes Vortriebsmittel und ein zweites Vortriebsmittel auf, welche zur Kraftübertragung miteinander wechselwirken. Das erste Vortriebsmittel und das zweite Vortriebsmittel können auf unterschiedliche Arten ausgestaltet sein um miteinander zu wechselwirken. Beispielsweise kann eines der beiden Vortriebsmittel mit Vertiefungen ausgestattet sein, welche im Zusammenspiel mit Vorhebungen des anderen Vortriebsmittels für eine Bewegungsübertragung sorgen.

Eine weitere Ausgestaltungsform der Vorrichtung ist dadurch gekennzeichnet, dass die Getriebeeinheit ein zahnstangenartig ausgebildetes erstes Vortriebsmittel und ein wenigstens partiell ritzelartig ausgestaltetes zweites Vortriebsmittel umfasst. Unter einem Vortriebsmittel ist ein bewegliches Bauteil der Getriebeeinheit zu verstehen, welches mit einem zweiten beweglichen Bauteil der Getriebeeinheit in Kontakt steht, derart, dass eine gerichtete Bewegung des einen Bauteils in eine gerichtete Bewegung des anderen Bauteils überführt wird. Die Vortriebsmittel wirken dabei derart zusammen, dass aus einer rotatorischen Bewegung des zweiten Vortriebsmittels eine lineare Bewegung des ersten Vortriebsmittels resultiert.

In einer ersten Ausgestaltungsform ist das erste Vortriebsmittel form- und/oder kraftschlüssig mit dem Kolben oder dem Zylinder des Kolben-Zylinder-Systems verbunden. In einer weiteren Ausgestaltungsform ist das erste Vortriebsmittel und der Kolben oder der Zylinder des Kolben-Zylinder-Systems einstückig ausgestaltet.

Bevorzugt ist, dass das erste Vortriebsmittel zahnstangenartig und das zweite Vortriebsmittel wenigstens partiell ritzelartig ausgebildet ist. Ein Vorteil eines zahnstangenartig ausgebildeten ersten Vortriebsmittels ist die unmittelbare Übertragung der Bewegungsrichtung der Zahnstange in eine axiale Bewegung des Kolbens und somit des Kolbens zu dem Kolben-Zylinder-System. Somit geht bei der Übertragung keine Krafteinwirkung des Anwenders verloren. Zudem ist die Getriebeeinheit auf diese Weise durch die Vermeidung von möglicherweise zusätzlich benötigten Getriebeeinheitsteilen möglichst störungssicher zu betreiben. Letzteres ist gerade bei schneller Förderung der Monomerflüssigkeit unter erhöhtem Förderdruck von Vorteil. Die wenigstens partiell ritzelartige Ausgestaltung des zweiten Vortriebsmittels dient der Übertragung einer rotatorischen Bewegung auf das erste Vortriebsmittel. Dabei sorgen die Ritzel für eine unmittelbare und anwendungssichere Kraftübertragung auf das erste Vortriebsmittel.

Eine weitere Ausgestaltungsform der Vorrichtung ist dadurch gekennzeichnet, dass das Antriebselement derart mit dem Zylinder verbunden ist, dass eine rotatorische Bewegung des Antriebselements in einer linearen Bewegung des Zylinders resultiert. Das Antriebselement kann im Allgemeinen auf zwei Weisen mit dem Kolben-Zylinder-System verbunden sein. In einer ersten Ausgestaltungsform ist das Antriebselement form- und/oder kraftschlüssig mit dem Kolben verbunden oder Antriebselement und Kolben sind einstückig ausgestaltet. In einer, bevorzugten, zweiten Ausgestaltungsform ist das Antriebselement mit dem Zylinder form- und/oder kraftschlüssig verbunden oder Antriebselement und Zylinder sind einstückig ausgestaltet.

Eine weitere Ausgestaltungform der Vorrichtung ist dadurch gekennzeichnet, dass das Antriebselement hebelartig ausgestaltet und an der Außenfläche des zweiten Behältnisses angeordnet ist, insbesondere, dass das Antriebselement um eine Achse drehbar gelagert ist, bevorzugt um wenigstens 90 Grad, insbesondere wenigstens 60 Grad drehbar gelagert ist, insbesondere dass die Achse im Wesentlichen rechtwinklig zu einer Längsachse der Vorrichtung angeordnet ist. Das Antriebselement kann an unterschiedlichen Stellen der Vorrichtung angebracht oder mit der Vorrichtung verbindbar sein. Bevorzugt ist das Antriebselement an einer, vorzugsweise nicht auf der Längsachse des zweiten Behältnisses liegenden, Außenfläche des zweiten Behältnisses angebracht. Ein Vorteil ist, dass so der Krafteintrag des Antriebselements auf das Kolben-Zylinder-System auf kurzem Wege und ohne zusätzliche Bauteile ermöglicht wird. Zudem ist so eine möglichst kompakte Bauform der Vorrichtung gegeben, was sich sowohl auf den geringeren Platzbedarf als auch auf die sicherere Verwendung durch den Anwender auswirkt. Das Antriebselement kann auf unterschiedliche Weisen ausgestaltet sein. Beispielsweise kann das Antriebselement händisch, hydraulisch oder pneumatisch betrieben werden. Vorzugsweise wird das Antriebselement durch Krafteintrag durch den Anwender der Vorrichtung betrieben. Der Krafteintrag durch den Anwender kann auf unterschiedliche Arten auf das Antriebselement übertragen werden. Beispielsweise kann der Krafteintrag durch einen Drehknopf oder ein Drehrad bewirkt werden. Vorzugsweise ist das Antriebselement hebelartig ausgestaltet. Ein Vorteil einer hebelartigen Ausgestaltung ist, dass der Kraftaufwand für den Anwender durch die Ausnutzung des Hebelprinzips verringert wird. Ein weiterer Vorteil ist, dass eine hebelartige Ausgestaltung die Anzahl an beweglichen Bauteilen, und damit die Fehleranfälligkeit des Antriebselements, reduziert.

Damit bei einer hebelartigen Ausgestaltung des Antriebselements das Hebelprinzip voll ausgenenutzt werden kann, ist das Antriebselement vorzugsweise um eine Achse drehbar gelagert. Weiter bevorzugt ist, dass für einen maximalen Kraftübertrag des Anwenders, das Antriebselement vorzugsweise um eine Achse drehbar gelagert ist, welche im Wesentlichen rechtwinklig zu einer Längsachse der Vorrichtung angeordnet ist.

Das Antriebselement kann vollständig frei um eine Achse drehbar gelagert sein. Aus Gründen einer kompakten Bauweise und einfachen Handhabbarkeit der Vorrichtung, ist das Antriebselement vorzugsweise um wenigstens 90 Grad, insbesondere um wenigstens 60 Grad drehbar gelagert.

Eine weitere Ausgestaltungsform der Vorrichtung ist dadurch gekennzeichnet, dass der Zylinder axial in dem zweiten Innenraum bewegbar ist.

Eine weitere Ausgestaltungsform der Vorrichtung ist dadurch gekennzeichnet, dass der Kolben form- und/oder kraftschlüssig mit dem zweiten Behältnis verbunden ist. Das Kolben-Zylinder-System kann auf unterschiedliche Weisen zum Fördern der Monomerflüssigkeit in den ersten Innenraum ausgestaltet sein. In einer ersten Ausgestaltungsform kann der Zylinder form- und/oder kraftschlüssig mit der Vorrichtung verbunden sein und der Kolben mittels des Antriebselements angetrieben werden. In einer weiteren, bevorzugten, Ausgestaltungsform ist der Kolben form- und/oder kraftschlüssig mit der Vorrichtung, insbesondere bevorzugt mit dem zweiten Behältnis der Vorrichtung verbunden, und der Zylinder wird durch das Antriebsmittel bewegt.

Eine weitere Ausgestaltungsform der erfindungsgemäßen Vorrichtung ist dadurch gekennzeichnet, dass das Antriebselement derart mit dem Zylinder verbunden ist, dass eine rotatorische Bewegung des Antriebselements um die Achse in einer linearen Bewegung des Zylinders resultiert.

In einer Ausgestaltungsform weist die Vorrichtung, bevorzugt das erste Behältnis, eine mechanische Mischeinrichtung zum Durchmischen des Knochenzementpulvers und der Monomerflüssigkeit auf. Beispielsweise kann die Mischeinrichtung aus einem axial in dem ersten Behältnis beweglichen Mischer bestehen, welcher durch eine Mischstange von außerhalbe der Vorrichtung bedient werden kann.

Eine weitere Ausgestaltungsform der Vorrichtung ist dadurch gekennzeichnet, dass die Vorrichtung, insbesondere das erste Behältnis, mischeinrichtungsfrei ist.. Ein Vorteil einer mischeinrichtungsfreien Vorrichtung ist, dass die Vorrichtung damit für den Anwender einfach und mit einer verringerten Gefahr von Fehlerquellen durch falsche Bedienung benutzt werden kann. Zudem verringern sich durch die geringere Bauteilanzahl die Herstellungskosten der Vorrichtung.

Eine weitere Ausgestaltung der erfindungsgemäßen Vorrichtung ist dadurch gekennzeichnet, dass das Lagerelement ein Gefäß mit der Monomerflüssigkeit beinhaltet. Erfindungsgemäß werden unter einem Gefäß alle Lagermöglichkeiten verstanden, welche die Monomerflüssigkeit hermetisch dicht und steril lagern können und durch manuelle Krafteinwirkung zerstörbar sind. Beispiele für Gefäße sind Glasampullen, Kunststoffampullen und Kunststoffbeutel. Aufgrund der leichten Sterilisierbarkeit und guten Öffenbarkeit durch manuelle Krafteinwirkung sind Glasampullen bevorzugt.

Eine weitere Ausgestaltung der erfindungsgemäßen Vorrichtung ist dadurch gekennzeichnet, dass das Lagerelement eine Öffnungsvorrichtung für das Gefäß aufweist, insbesondere dass die Öffnungsvorrichtung ein Anstechdorn oder eine Bruchkante ist. Erfindungsgemäß ist unter einer Öffnungsvorrichtung eine Vorrichtung zu verstehen, welche dazu geeignet ist, die strukturelle Integrität des Gefäßes zu zerstören und damit zu öffnen. Die Ausgestaltung der Öffnungsvorrichtung ist dabei in Abhängigkeit der strukturellen Integrität des Gefäßes zu wählen. Das Material der Öffnungsvorrichtung ist erfindungsgemäß dazu in der Lage, die strukturelle Integrität des Materials des Gefäßes zu zerstören. Beispielsweise kann das Öffnen des Gefäßes durch Einstechen, Einschneiden oder Aufbrechen erfolgen. Beispiele für Öffnungsvorrichtungen umfassen Anstechdorne, Nadeln, Kanülen, Schneidkanten und Bruchkanten. In einer bevorzugten Ausgestaltungsform ist das Gefäß eine Glasampulle und die Öffnungsvorrichtung ein Anstechdorn oder eine Bruchkante. Der Anstechdorn hat den Vorteil, dass er die Glasampulle punktuell öffnen kann, den Rest der Ampulle jedoch intakt lässt. Die Bruchkante hat den Vorteil, dass unter der Ausnutzung des Hebelprinzips eine spezifische Stelle der Glasampulle unter verminderter Kraftanstrengung des Anwenders aufgebrochen werden kann. Falls als Öffnungsvorrichtung eine Bruchkante eingesetzt wird, muss das Gefäß relativ zu einer Achse der Bruchkante drehbar gelagert sein, um geöffnet zu werden. In einer ersten Ausgestaltungsform kann dies dadurch erreicht werden, dass der dritte Innenraum des Lagerelements so groß ausgestaltet ist, dass das Gefäß innerhalb des dritten Lagerraums relativ zur Bruchkante drehbar gelagert ist. In einer weiteren, bevorzugten, Ausgestaltungsform, ist das Lagerelement selbst relativ zu einer Achse der Bruchkante drehbar gelagert. Eine Drehung des Lagerelements, in dem das Gefäß gelagert ist, um eine Achse der Bruchkante öffnet somit das Gefäß. Ein Vorteil der letztgenannten Ausgestaltungsform ist, dass die Verletzungsgefahr im Vergleich zu der ersten Ausgestaltungsform für den Anwender reduziert ist. Um eine Drehung um eine Achse der Bruchkante zu ermöglichen, kann das Lagerelement entlang der Drehachse ein Gelenk aufweisen oder das Lagerelement besteht, zumindest im Bereich der Drehachse, aus einem flexiblen Material, wie beispielsweise Kautschuk.

Eine weitere Ausgestaltungsform der erfindungsgemäßen Vorrichtung ist dadurch gekennzeichnet, dass in dem ersten Behältnis ein axial verschiebbarer Austragskolben angeordnet ist. Der Austragskolben dient dem Austragen des gemischten Knochenzements aus dem ersten Behältnis aus einer Austragsvorrichtung. Der Austragskolben ist erfindungsgemäß an dem zweiten Behältnis zugewandten Ende des ersten Behältnisses angeordnet. Die Austragsvorrichtung ist erfindungsgemäß an einem dem Austragskolben entgegengesetzten Ende des ersten Behältnisses angebracht. Das Knochenzementpulver ist dabei zwischen Austragsvorrichtung und Austragskolben angeordnet. Eine axiale Bewegung des Austragskolben in Richtung der Austragsvorrichtung treibt somit den Knochenzement nach dem Mischen der Ausgangskomponenten aus dem ersten Behältnis aus. Die Lage des Austragskolbens innerhalb des ersten Behältnisses bedingt, dass die Monomerflüssigkeit beim Fördern aus dem zweiten Innenraum in den ersten Innenraum den Austragskolben passieren muss, um mit dem Knochenzementpulver in Kontakt zu kommen. In einer ersten Ausgestaltungsform umspült die Monomerflüssigkeit den Austragskolben, um mit dem Knochenzementpulver in Kontakt zu kommen. Beispielsweise kann die Monomerflüssigkeit dabei durch, im Wesentlichen axial verlaufende, Vertiefungen in der Innenwand des ersten Behältnisses und/oder in der Außenfläche des Austragskolbens in Richtung des Knochenzementpulvers gefördert werden. In einer weiteren Ausgestaltungsform weist der Austragskolben mindestens eine, im Wesentlichen axial verlaufende, Durchführung auf, durch welche die Monomerflüssigkeit in Richtung des Knochenzementpulvers gefördert werden kann. Vorzugsweise ist die mindestens eine Durchführung derart gestaltet, dass diese für Gase und Flüssigkeiten durchlässig und für Feststoffe undurchlässig ausgestaltet ist. Beispielsweise weist die mindestens eine Durchführung ein Sieb oder eine Porenscheibe auf. Auf diese Weise wird ein Fördern der Monomerflüssigkeit vom zweiten Innenraum in den ersten Innenraum ermöglicht und gleichzeitig ein unerwünschtes Fördern des Knochenzementpulvers und/oder des fertig gemischten Knochenzements in den zweiten Innenraum unterbunden. Die Durchführung kann dabei als eine Verlängerung des zweiten Leitungsmittels ausgestaltet sein. In einer Ausgestaltungsform sind das erste Behältnis und das zweite Behältnis stoffschlüssig miteinander verbunden oder erstes Behältnis und zweites Behältnis sind einstückig ausgestaltet.

Eine weitere Ausgestaltungsform der erfindungsgemäßen Vorrichtung ist dadurch gekennzeichnet, dass das erste Behältnis ein erstes Anschlussmittel und das zweite Behältnis ein zweites Anschlussmittel aufweisen, um das erste Behältnis und das zweite Behältnis, insbesondere reversibel, zu verbinden, insbesondere reversibel form- und/oder kraftschlüssig zu verbinden. In einer ersten Ausgestaltungsform formen das erste Anschlussmittel und das zweite Anschlussmittel einen Bajonettverschluss. In einer weiteren Ausgestaltungsform ist das erste Anschlussmittel als ein Innen- oder Außengewinde und das zweite Anschlussmittel als eine dazu passenden Außen- oder Innengewinde ausgestaltet, derart, dass das Innengewinde und das Außengewinde form- und oder kraftschlüssig miteinander verbindbar sind.

Ein Vorteil einer reversibel lösbaren kraft- und/oder formschlüssigen Verbindung zwischen erstem Behältnis und zweitem Behältnis liegt darin, dass nach erfolgtem Mischen des Knochenzementpulvers und der Monomerflüssigkeit das erste Behältnis vom zweiten Behältnis abgetrennt werden kann, um den gemischten Knochenzement mittels einer Austragvorrichtung, wie beispielsweise einer Austragspistole, aus dem ersten Behältnis auszutragen.

Ein weiterer Gegenstand der Erfindung betrifft ein Verfahren zur Bereitstellung eines Knochenzement aus zwei Ausgangskomponenten mittels einer Vorrichtung gemäß einer der vorangehenden Ausgestaltungsformen, zumindest umfassend die folgenden sequenziellen Teilschritte:
a) Überführen des Kolben-Zylinder-Systems in die erste Position,
b) Fördern der Monomerflüssigkeit mittels der fluidleitenden Verbindung in das Fördervolumen,
c) Überführen des Kolben-Zylinder-Systems aus der ersten Position in die zweite Position,
d) Verschließen der fluidleitenden Verbindung zwischen dem zweiten Leitungsmittel und dem Fördervolumen,
e) Fördern der Monomerflüssigkeit in den ersten Innenraum,
f) Ausbildung des Knochenzements aus Knochenzementpulver und Monomerflüssigkeit.

Eine weitere Ausgestaltungsform des erfindungsgemäßen Verfahrens ist dadurch gekennzeichnet, dass die Ausbildung des Knochenzements aus Knochenzementpulver und Monomerflüssigkeit ohne mechanische Einwirkung erfolgt. Nach erfolgtem Fördern der Monomerflüssigkeit aus dem zweiten Innenraum in den ersten Innenraum erfolgt die Ausbildung des Knochenzements. In einer ersten Ausgestaltungsform wird zur Ausbildung des Knochenzements aktiv ein mechanischer Mischvorgang durch den Anwender ausgeführt. Beispielsweise betätigt der Anwender einen Mischer, der mittels einer Mischstange aktiv von Außerhalb der Vorrichtung angetrieben werden kann. Der Mischer kann dabei beispielsweise periodisch entlang einer Längsachse innerhalb des ersten Behältnisses hin und her bewegt werden, beispielsweise bei gleichzeitiger periodischer Drehung entlang der Längsachse der Mischstange. In einer weiteren, bevorzugten Ausgestaltungsform des Verfahrens, führt der Anwender keinen aktiven mechanischen Mischvorgang der zwei Ausgangskomponenten aus.

Die erfindungsgemäße Vorrichtung ist dadurch gekennzeichnet, dass diese Knochenzemente aus zwei Ausgangskomponenten bereitstellt. Erfindungsgemäß wird unter einem Knochenzement eine Substanz verstanden, die geeignet ist im Bereich der Medizintechnik eine stabile Verbindung zwischen künstlichen Gelenken, wie beispielsweise Hüft- und Kniegelenken, und Knochenmaterial zu erstellen. Bevorzugt handelt es sich bei Knochenzementen um Polymethylmethacrylat-Knochenzemente (PMMA Knochenzemente). PMMA-Knochenzemente kommen schon lange in medizinischen Anwendungen zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einem Knochenzementpulver als erster Ausgangskomponente und einer Monomerflüssigkeit als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer Knochenzement, der auch als Knochenzementteig bezeichnet wird. Dabei wird eine Polymerisation des Monomers durch Radikale eingeleitet. Mit fortschreitender Polymerisation des Monomers erhöht sich die Viskosität des Knochenzements, bis dieser vollständig aushärtet.

Erfindungsgemäß wird unter einem Knochenzementpulver ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulvers zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver mindestens ein partikuläres Polymethylmethacrylat und/oder ein partikuläres Polymethylmethacrylat-Copolymer, einen Initiator und einen Röntgenopaker oder besteht aus diesen Komponenten. Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotikum oder besteht aus diesen Komponenten.

Erfindungsgemäß kann die Partikelgröße des partikulären Polymethylmethacrylat und/oder des partikulären Polymethylmethacrylat-Copolymers des Knochenzementpulvers der Siebfraktion kleiner 150 µm, bevorzugt kleiner 100 µm, entsprechen.

Erfindungsgemäß kann das hydrophile Additiv partikulär und/oder faserförmig ausgestaltet sein. In einer weiteren Ausgestaltungsform kann das hydrophile Additiv schwerlöslich, bevorzugt unlöslich, in Methylmethacrylat sein. In einer weiteren Ausgestaltungsform kann das hydrophile Additiv ein Aufsaugvermögen von mindestens 0,6 g Methylmethacrylat pro Gramm hydrophiles Additiv besitzen. In einer weiteren Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Erfindungsgemäß kann der Initiator Dibenzoylperoxid enthalten oder aus Dibenzoylperoxid bestehen.

Erfindungsgemäß versteht man unter einem Röntgenopaker eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen.

Erfindungsgemäß kann die pharmazeutisch aktive Substanz ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind Gentamicin, Clindamycin und Vancomycin.

Erfindungsgemäß kann die Monomerflüssigkeit das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin.

### Beispiele

Die Erfindung wird im Folgenden durch Beispiele weiter beispielhaft illustriert. Die Erfindung ist nicht auf die Beispiele beschränkt.

### Figuren

### Es zeigen

- Fig. 1: eine schematische Zeichnung einer Seitenansicht einer Vorrichtung zum Bereitstellen eines Knochenzements aus zwei Ausgangskomponenten,
- Fig. 2: die Vorrichtung aus Fig. 1 um 180° gedreht,
- Fig. 3: die Vorrichtung aus Fig. 2 aus in einer perspektivischen Seitenansicht,
- Fig. 4: die Vorrichtung aus Fig. 2 als schematischer Querschnitt, und
- Fig. 5: ein Flussdiagramm (Verfahren zur Bereitstellung eines Knochenzements aus zwei Ausgangskomponenten).

### Beschreibung der Figuren

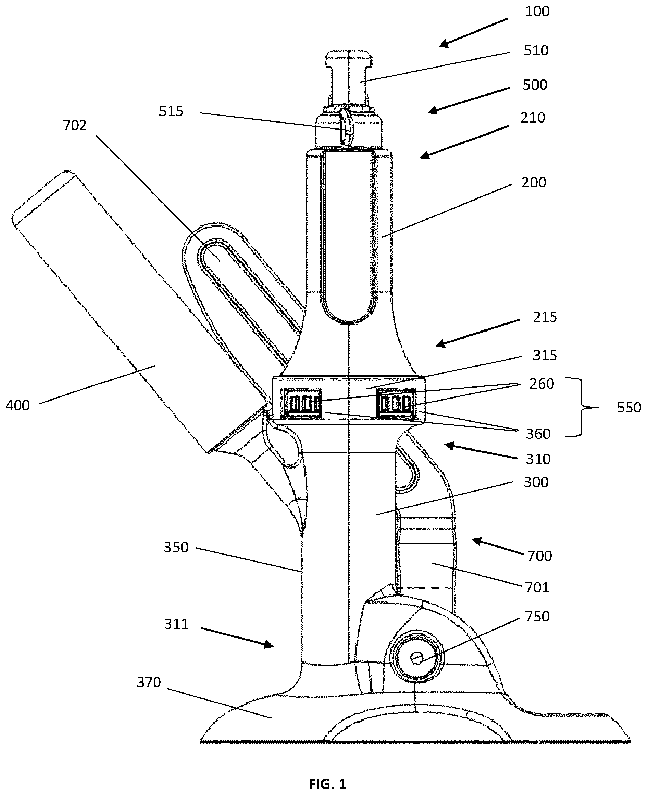

**Figur 1** zeigt eine Vorrichtung 100 zum Bereitstellen eines Knochenzements aus zwei Ausgangskomponenten. Die Vorrichtung 100 ist einstückig, aber aus mehreren Bauteilen aufgebaut. Die Vorrichtung 100 weist ein rohrartiges erstes Behältnis 200 auf. Das erste Behältnis 200 weist an einem ersten Ende 210 eine Austragsvorrichtung 500 und einen Verschluss 510 auf. Der Verschluss 510 dient dazu, ein ungewolltes Austragen des Knochenzements aus der Austragsvorrichtung 500 zu verhindern. Der Verschluss 510 umfasst mindestens einen Drehflügel 515, mit dem der Verschluss 510 durch eine Drehbewegung vom ersten Behältnis 200 gelöst werden kann. In einer weiteren Ausgestaltungsform lässt sich der Verschluss 510 vom ersten Behältnis 200 lösen, indem der Anwender nicht an einem Drehflügel 515, sondern an einem Stift, einer Öse oder an einer Außenwand des Verschlusses 510 selbst dreht. In einer weiteren Ausgestaltungsform ist der Verschluss 510 nicht durch Abdrehen, sondern beispielsweise durch Abziehen von dem ersten Behältnis 200 zu lösen.

Das erste Behältnis 200 ist an einem zweiten Ende 215 mit einem zweiten Behältnis 300 verbunden. Das erste Behältnis 200 und das zweite Behältnis 300 sind axial miteinander verbunden. Das erste Behältnis 200 und das zweite Behältnis 300 sind gemeinsam auf einer Mittelachse der Vorrichtung 100 angeordnet. Das erste Behältnis 200 weist an dem zweiten Ende 215 ein erstes Anschlussmittel 260 und das zweite Behältnis 300 weist an einem ersten Ende 310 ein zweites Anschlussmittel 360 auf. Das erste Anschlussmittel 260 ist in Form von mindestens einer Nase und das zweite Anschlussmittel 360 ist in Form von mindestens einer Aussparung ausgeformt. Die mindestens eine Aussparung befindet sich in einem Flanschring 315, welcher das zweite Ende 215 des ersten Behältnisses 200 zumindest teilweise umschließt. Das erste Anschlussmittel 260 in Form von mindestens einer Nase und das zweite Anschlussmittel 360 in Form von mindestens einer Aussparung bilden zusammen einen Bajonettverschluss 550.

Weitere Ausgestaltungsformen der erfindungsgemäßen Vorrichtung verbinden das erste Behältnis 200 und das zweite Behältnis 300 nicht über einen Bajonettverschluss 550, sondern beispielsweise über eine Gewindeverbindung, über eine Druckknopfverbindung und/oder über eine Klammerverbindung. Das erste Behältnis 200 und das zweite Behältnis 300 sind über eine lösbare Verbindung miteinander verbunden. Das erste Behältnis 200 kann von dem zweiten Behältnis 300 gelöst werden, um einen gemischten Knochenzement aus dem ersten Behältnis 200 auszutragen. Dazu kann das erste Behältnis 200 mittels des ersten Anschlussmittels 260 an eine Austragpistole angeschlossen werden, welche den Knochenzement aus der Austragsvorrichtung 500 drückt. Um den Knochenzement zielgenau an die gewünschte Position zu applizieren, kann es vorteilhaft sein, einen Applizierschnorchel vor dem Austragen des Knochenzements an der Austragsvorrichtung 500 anzubringen.

Das zweite Behältnis 300 ist rohrartig ausgebildet. Das zweite Behältnis weist an einem zweiten Ende 311 ein Standelement 370 auf. Das Standelement 370 dient dem stabilen Stand der Vorrichtung auf einer Unterfläche, wie beispielsweise einer Arbeitsplatte oder einem Tisch. Mittels des Standelements 370 kann der Anwender die Vorrichtung 100 auf einer Unterfläche abstellen, ohne dass die Vorrichtung 100 selbstständig umfällt. Des Weiteren dient das Standelement 370 dazu, die Vorrichtung 100 während der Verwendung in der richtigen räumlichen Ausrichtung zu halten und dem Anwender somit die Bedienung der Vorrichtung 100 zu erleichtern. In der gezeigten Ausgestaltungsform ist das Standelement 370 als flaches Bauteil ausgeformt, welches zumindest teilweise auf einer planen Unterfläche anliegen kann. In einer weiteren Ausgestaltungsform kann das Standelement 370 zwei oder mehr Ständer umfassen, mit denen die Vorrichtung 100 auf einer Unterfläche abgestellt werden kann.
Das zweite Behältnis 300 weist an einer Außenfläche 350 ein Lagerelement 400 auf. Das Lagerelement 400 ist rohrartig ausgestaltet. Das Lagerelement 400 ist derart ausgestaltet, dass es eine Glasampulle mit einer Monomerflüssigkeit aufnehmen kann.

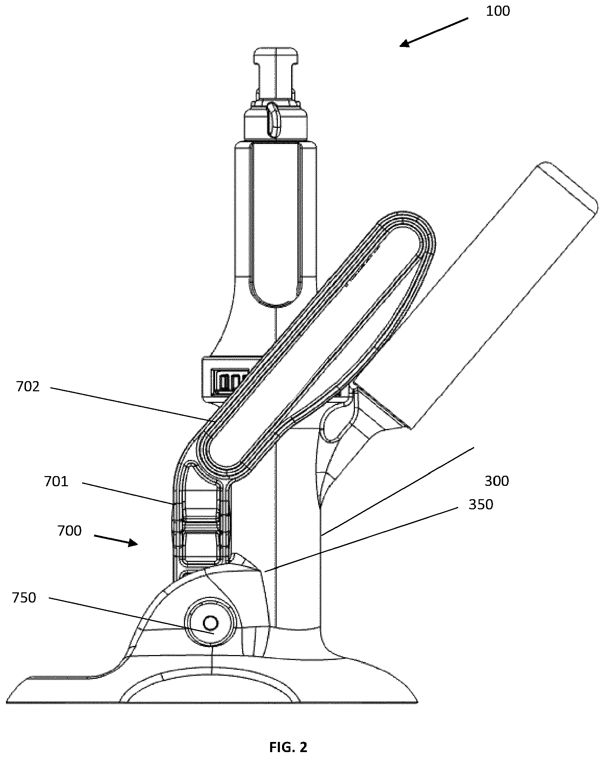

**Figur 2** zeigt die Vorrichtung 100 aus Figur 1 um 180° gedreht. Die Vorrichtung 100 umfasst ein Antriebselement 700. Das Antriebselement 700 ist an der Außenfläche 350 des zweiten Behältnisses 300 angeordnet. Die Vorrichtung 100 weist eine Achse 750 auf, um welche das Antriebselement 700 zumindest teilweise gedreht werden kann.
Das Antriebselement 700 ist hebelartig ausgestaltet. Das Antriebselement 700 weist einen ersten Teil 701 und einen zweiten Teil 702 auf, wobei der erste Teil 701 und der zweite Teil 702 einstückig ausgestaltet sind. Der erste Teil 701 erstreckt sich zumindest teilweise parallel zu einer Längsachse des zweiten Behältnisses 300. Der zweite Teil 702 ist zum ersten Teil 701 abgewinkelt. Der zweite Teil 702 ist in Form eines Griffs ausgestaltet. Die gebogene Form des Antriebselements 700 erlaubt es dem Anwender, das Antriebselement 700 um die Achse 750 derart zu drehen, dass der erste Teil 701 des Antriebselements um bis zu 90° gedreht werden kann, ohne dass die Hände des Anwenders die Unterfläche, auf der die Vorrichtung 100 steht, berühren. Somit ist dem Anwender die Verwendung der Vorrichtung 100 erleichtert. In einer weiteren, nicht gezeigten, Ausgestaltungsform ist das Antriebselement 700 nicht gebogen, sondern geradlinig. In weiteren, nicht gezeigten, Ausgestaltungsformen weist die Vorrichtung 100 anstelle eines hebelartigen Antriebselements 700 eine andere Struktur auf, die um die Achse 750 gedreht werden kann. Beispielsweise kann das Antriebselement 700 als Drehrad ausgestaltet sein.

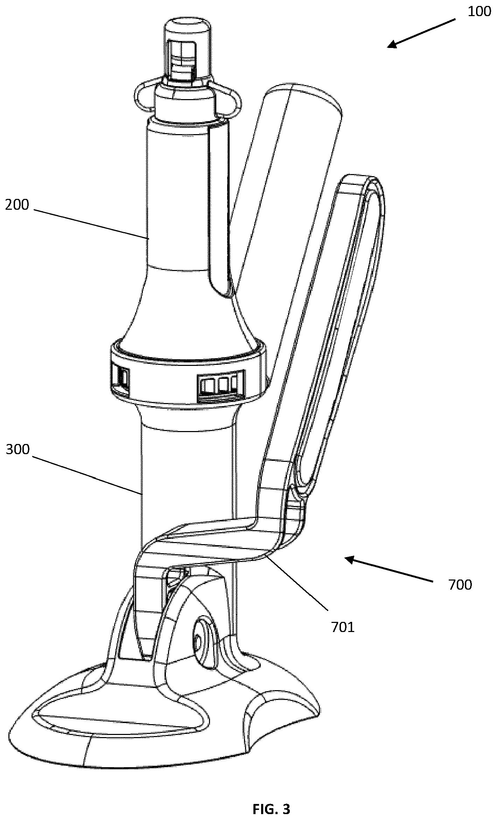

**Figur 3** zeigt die Vorrichtung 100 aus Figur 1 und 2 in einer perspektivischen Darstellung. In der Darstellung ist zu erkennen, dass der erste Teil 701 des Antriebselements 700 abgewinkelt ist. Ein Vorteil ist, dass dadurch die Drehbewegung des Antriebselements 700 nicht räumlich durch das erste Behältnis 200 und/oder das zweite Behältnis 300 eingeschränkt ist. Ein weiterer Vorteil ist, dass das Antriebselement 700 dadurch leichter von einem Anwender bedient werden kann.

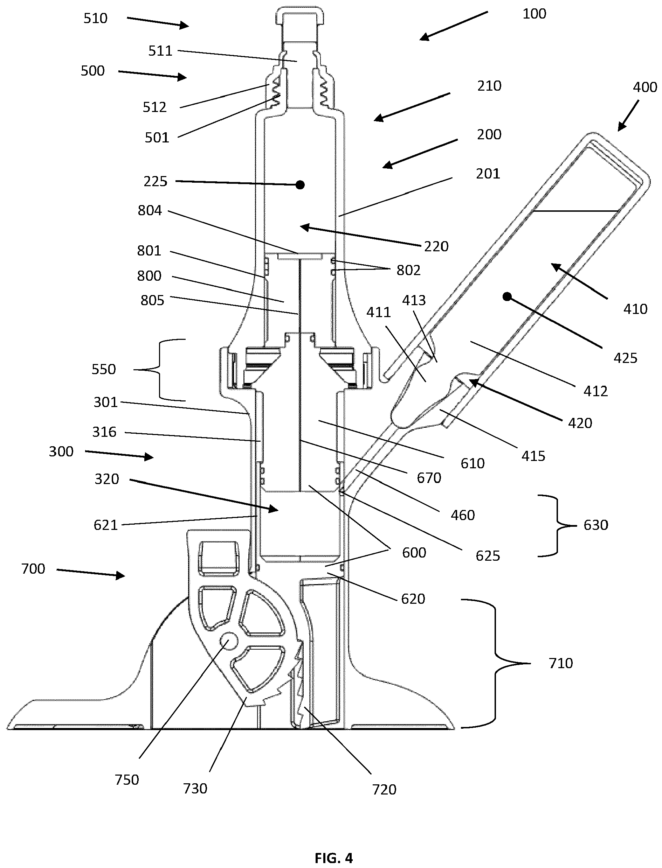

**Figur 4** zeigt die Vorrichtung 100 aus Figur 1, 2 und 3 als schematischen Schnittzeichnung. Die Vorrichtung 100 ist einstückig, aber aus mehreren Bauteilen aufgebaut. Die Vorrichtung 100 weist das rohrförmige erste Behältnis 200 auf. Das erste Behältnis 200 weist einen ersten Innenraum 220 auf. Der erste Innenraum 220 ist von einer ersten Behältniswand 201 des ersten Behältnisses 200 umgeben. In dem ersten Innenraum 220 ist ein Knochenzementpulver 225 gelagert. Der gesamte erste Innenraum 220 ist mit dem Knochenzementpulver 225 ausgefüllt. Das Knochenzementpulver 225 ist so im ersten Innenraum 220 verdichtet, dass das Knochenzementpulver 225 nicht fließfähig ist. In den Zwischenräumen des Knochenzementpulvers 225 befindet sich ein Gas, wie beispielsweise Luft, Stickstoff oder Argon.

Das erste Behältnis 200 weist am ersten Ende 210 die Austragsvorrichtung 500 mit einem Außengewinde 501 auf. Die Austragsvorrichtung 500 ist durch den Verschluss 510 verschlossen. Der Verschluss 510 umfasst einen Verschlussstift 511 und ein Verschlussinnengewinde 512. Das Verschlussinnengewinde 512 wirkt form- und/oder kraftschlüssig mit dem Außengewinde 501 der Austragsvorrichtung 500 zusammen. Der Verschlussstift 511 verschließt die Austragsvorrichtung 500. Der Verschlussstift 511 verhindert ein ungewolltes Austragen des Knochenzementpulvers 225 aus der Vorrichtung 100. Der Verschlussstift 511 ist für Gase und optional auch für eine Monomerflüssigkeit 425 durchlässig ausgestaltet. Bei der Ausdehnung des fertig gemischten Knochenzements wird der Verschlussstift 511 teilweise aus der Austragsvorrichtung 500 ausgetrieben. Dies zeigt dem Anwender eine ordnungsgemäße Funktion der Vorrichtung 100 und eine vollständige Benetzung des Knochenzementpulvers 225 durch die Monomerflüssigkeit 425 an.

Durch die Vermischung von Knochenzementpulvers 225 und Monomerflüssigkeit 425 bildet sich ein Knochenzement aus. Das Zusammenführen von Knochenzementpulvers 225 und Monomerflüssigkeit 425 resultiert in einer Quellung des Knochenzementpulvers 225.

Das erste Behältnis 200 weist einen Austragskolben 800 auf. Der Austragskolben 800 dient dem Austragen des fertig gemischten Knochenzements aus dem ersten Behältnis 200. Der Austragskolben 800 grenzt mit einer Außenfläche 801 an die Behältniswand 201 des ersten Behältnisses 200. Der Austragskolben 800 weist an der Außenfläche 801 zwei Dichtungsringe 802 auf. Die Dichtungsringe 802 gewährleisten einen Kompromiss zwischen fester Arretierung und leichter Verschiebbarkeit des Austragskolben 800. Das erste Behältnis 200 ist über den Bajonettverschluss 550 mit dem zweiten Behältnis 300 verbunden.

Das zweite Behältnis 300 weist einen zweiten Innenraum 320 auf. In dem zweiten Innenraum 320 ist ein Kolben-Zylinder-System 600 angeordnet. Das Kolben-Zylinder-System 600 weist einen Kolben 610 und einen Zylinder 620 auf. Der Zylinder 620 und der Kolben 610 sind relativ zueinander axial beweglich. In der gezeigten Ausgestaltungsform ist der Kolben 610 fest mit dem zweiten Behältnis 300 verbunden und der Zylinder 620 ist axial im zweiten Behältnis 300 beweglich ausgestaltet. Kolben 610 und Zylinder 620 bilden zusammen ein Fördervolumen 630 aus. Der Kolben 610 weist einen Außenumfang auf, welcher im Wesentlichen einem Innenumfang des Zylinders 620 entspricht. Der Zylinder 620 umgibt einen Hohlraum, welcher im Wesentlichen dem Volumen des Kolbens 610 entspricht. Der vom Zylinder 620 gebildete Hohlraum kann den Kolben 610 durch relatives aufeinander zubewegen von Zylinder 620 und Kolben 610 aufnehmen. Das Kolben-Zylinder-System 600 dient dem Fördern der Monomerflüssigkeit 425 aus dem Fördervolumen 630 in den ersten Innenraum 220. Die Monomerflüssigkeit 425 kann durch ein relatives aufeinander zubewegen von Kolben 610 und Zylinder 620 in den ersten Innenraum 220 verbracht werden. In der gezeigten Ausgestaltungsform ist der Zylinder 620 relativ zum Kolben 610 verschiebbar. Um in der gezeigten Ausgestaltungsform eine Bewegung des Zylinders 620 relativ zum Kolben 610 zu ermöglichen, befindet sich zwischen Kolben 610 und einer Behältniswand 301 des zweiten Behältnisses 300 ein Hohlraum 316. Der Hohlraum 316 ist derart ausgeformt, dass er den Zylinder 620 zumindest teilweise aufnehmen kann.

Um die Monomerflüssigkeit 425 aus dem Fördervolumen 630 in den ersten Innenraum 220 zu verbringen, ist das Fördervolumen 630 über ein erstes Leitungsmittel 670 fluidleitend mit dem ersten Innenraum 220 verbunden. Je nach räumlicher Lage von Kolben 610, Zylinder 620 und ersten Behältnis 200, weist entweder der Kolben 610 oder der Zylinder 620 das erste Leitungsmittel 670 auf. In einer ersten, nicht gezeigten, Ausgestaltungsform, weist der Zylinder 620 das erste Leitungsmittel 670 auf. In einer zweiten, gezeigten, Ausgestaltungsform, weist der Kolben 610 das erste Leitungsmittel 670 auf. In der zweiten Ausgestaltungsform ist das erste Leitungsmittel 670 als rohrartige Verbindung auf der gesamten Längsachse des Kolbens 610 ausgestaltet. Das erste Leitungsmittel 670 kann unterschiedliche Querschnittsgeometrien, wie beispielsweise quadratisch, elliptisch, dreieckig oder rechteckig aufweisen. Aufgrund der einfachen Herstellung hat das erste Leitungsmittel 670 bevorzugt eine kreisförmige Querschnittsgeometrie. Das erste Leitungsmittel 670 kann unterschiedliche Querschnittsdurchmesser aufweisen. Um einen guten Kompromiss von schneller Förderung bei großem Durchmesser und hohem Förderdruck bei kleinem Durchmesser zu erzielen, hat die Querschnittsfläche des ersten Leitungsmittels 670 beispielsweise einen Durchmesser von 0,5 mm² und 2 mm².

In der gezeigten Ausgestaltungsform, weist der Austragskolben 800 eine Durchführung 805 als Fortsetzung des ersten Leitungsmittels 670 auf. Dazu sind Kolben 610 und Austragskolben 800 derart angeordnet, dass sie sich im Bereich ihrer jeweiligen Längsachsen kontaktieren. Um ein unbeabsichtigtes Verbringen des Knochenzementpulver 225 aus dem ersten Innenraum 220 in das Fördervolumen 630 zu unterbinden, ist die Durchführung 805 des Austragskolben 800, als Forstsetzung des ersten Leitungsmittels 670, an einem dem Knochenzementpulver 225 zugewandten Ende mit einem Dichtungselement 804 versehen. Das Dichtungselement 804 ist derart ausgestaltet, dass es für Gase und Flüssigkeiten durchlässig, für Feststoffe hingegen undurchlässig ist. Auf diese Weise kann die Monomerflüssigkeit 425 aus dem Fördervolumen 630 in den ersten Innenraum 220 gepumpt werden, ein Rückfließen des Knochenzementpulvers 225 und/oder des gemischten Knochenzements ist hingegen unterbunden. Als Dichtungselement 804 kann jede Struktur dienen, welche die oben beschriebene Funktionalität aufweist. Beispielsweise kann das Dichtungselement 804 eine Porenscheibe, ein Sieb, ein Netz, eine Gitterstruktur oder eine Membran aufweisen oder daraus bestehen.

An der Außenfläche 350 des zweiten Behältnisses 300 ist das Lagerelement 400, aufweisend einen dritten Innenraum 420, angebracht. Das Lagerelement 400 ist nicht auf einer Längsachse des zweiten Behältnisses 300 angeordnet. Der dritte Innenraum 420 dient der Lagerung der Monomerflüssigkeit 425. In einer ersten, nicht gezeigten, Ausgestaltungsform ist die Monomerflüssigkeit 425 derart in dem dritten Innenraum 420 gelagert, dass die Wände des Lagerelements 400 mit der Monomerflüssigkeit in Berührung kommen. In einer bevorzugten, gezeigten, Ausgestaltungsform, befindet sich die Monomerflüssigkeit 425 in einem Gefäß 410, welches in dem dritten Innenraum 420 eingebracht ist. Die Verwendung von Gefäßen 410 ist bevorzugt, da so ein leichter und spontaner Austausch von Monomerflüssigkeit 425 unterschiedlicher chemischer Zusammensetzung und/oder Menge im Zuge einer laufenden Operation gewährleistet ist. Somit ist die Vorrichtung 100 variabler einsetzbar. Unter einem Gefäß 410 beinhaltend die Monomerflüssigkeit werden alle Lagermöglichkeiten verstanden, welche die Monomerflüssigkeit 425 hermetisch dicht und steril lagern können und welche durch manuelle Krafteinwirkung geöffnet werden können. In einer ersten, nicht gezeigten, Ausgestaltungsform handelt es sich bei dem Gefäß 410 um einen Kunststoffbeutel. In einer bevorzugten, gezeigten, Ausgestaltungsform handelt es sich bei dem Gefäß 410 um eine Glasampulle. Glasampullen sind bevorzugt, da sie leicht sterilisierbar sind und leicht durch manuelle Krafteinwirkung geöffnet werden können.

Das Lagerelement 400 weist eine Öffnungsvorrichtung 415 auf. Die Öffnungsvorrichtung 415 ist dazu geeignet, das Gefäß 410 mechanisch zu öffnen, damit die Monomerflüssigkeit 425 aus dem Inneren des Gefäßes 410 ausfließen kann. Die Öffnungsvorrichtung 425 ist dabei nach Art des zu öffnenden Gefäßes 410 zu wählen. Ist das Gefäß 410 beispielsweise ein Kunststoffbeutel, so kann die Öffnungsvorrichtung eine Schneidkante, eine Nadel oder eine Kanüle sein. In der gezeigten Ausgestaltungsform ist das Gefäß 410 eine Glasampulle. Geeignete Öffnungsvorrichtungen 415 für Glasampullen sind beispielsweise Anstechdorne, oder hammerähnliche Schlagelemente, mit denen die Glasampulle aufgebrochen werden kann. In einer bevorzugten, gezeigten, Ausgestaltungsform ist die Öffnungsvorrichtung 415 als Bruchkante ausgeformt. Die Bruchkante ist derart gestaltet, dass sie den Glasampullenkopf 411 des Gefäßes 410 aufnehmen kann, so dass eine Knickbewegung des Lagerelements 400, und damit des Glasammpullenkörpers 412, zu einem Aufbrechen des Glasampullenhals 413 führt. Dazu ist die Öffnungsvorrichtung 415 konisch geformt, wobei der Querschnittsverlauf im Wesentlichen an den Querschnittsverlauf des Ampullenkopfes 411 angepasst ist. Bevorzugt liegt allerdings keine exakte Anpassung der Öffnungsvorrichtung 415 in Form einer Bruchkante an den Ampullenkopf 411 vor, da sonst ein Umspüllen des Ampullenkopfs 411 durch die Monomerflüssigkeit, und damit ein Fließen in den zweiten Innenraum 320, unterbunden sein könnte.

In der gezeigten Ausgestaltungsform weist die Vorrichtung 100 ein zweites Leitungsmittel 460 zwischen Öffnungsvorrichtung 415 und zweitem Behältnis 300 auf, welches den dritten Innenraum 420 fluidleitend mit dem Fördervolumen 630 verbindet oder verbinden kann. Je nach Ausgestaltungsform des Gefäßes 410, und damit der Öffnungsvorrichtung 415, kann es sinnvoll sein, das zweite Leitungsmittel 460 zwar mit dem dritten Innenraum 420, aber nicht direkt mit der Öffnungsvorrichtung 415 zu verbinden. Das zweite Leitungsmittel 460 ermöglicht ein Fließen der Monomerflüssigkeit 425 aus dem dritten Innenraum 420 in das Fördervolumen 630 des Kolben-Zylinder-Systems 600. Das zweite Leitungsmittel 460 ist als rohrartige Verbindung zwischen drittem Innenraum 420 und Fördervolumen 630 ausgestaltet. Das zweite Leitungsmittel 460 kann unterschiedliche Querschnittsgeometrien, wie beispielsweise quadratisch, elliptisch, dreieckig oder rechteckig aufweisen. Aufgrund der einfachen Herstellung hat das zweite Leitungsmittel 460 bevorzugt eine kreisförmige Querschnittsgeometrie. Das zweite Leitungsmittel 460 schließt an eine lochartige Zylinderdurchführung 625 in einer Zylinderwand 621 des Zylinders 620 an, durch die die Monomerflüssigkeit 425 in das Fördervolumen 630 fließen kann. Die Zylinderdurchführung 625 und das zweite Leitungsmittel 460 weisen bevorzugt einen im Wesentlichen gleichen Querschnittsdurchmesser auf.

In einer ersten Position des Kolben-Zylinder-Systems 600 ist das zweite Leitungsmittel 460 fluidleitend mit dem Fördervolumen 630 verbunden. Die erste Position stellt somit eine Ausgangsposition der Vorrichtung 100 dar, in der die Monomerflüssigkeit 425 aus dem dritten Innenraum 420 in das Fördervolumen 630, vorzugsweise nach dem Öffnen des Gefäßes 410, fließen kann. Um im Zuge eines Förderns der Monomerflüssigkeit 425 aus dem Fördervolumen 630 in den ersten Innenraum 220 ein Rückfließen der Monomerflüssigkeit 425 in den dritten Innenraum 420 zu verhindern, ist das zweite Leitungsmittel 460 in einer zweiten Position des Kolben-Zylinder-Systems 600 nicht fluidleitend ausgestaltet. Der Wechsel von fluidleitend zu nicht-fluidleitend wird mittels der Zylinderdurchführung 625 bewirkt. Die Zylinderdurchführung 625 befindet sich in der ersten Position des Kolben-Zylinder-Systems 600 auf Höhe des zweiten Leitungsmittels 460, so dass die Monomerflüssigkeit 425 durch die Zylinderdurchführung 625 in das Fördervolumen 630 einfließen kann. In der zweiten Position des Kolben-Zylinder-Systems 600 ist der Zylinder 620, und damit auch die Zylinderdurchführung 625, relativ zur ersten Position nach oben, in Richtung des ersten Behältnisses 200, verschoben, so dass das zweite Leitungsmittel 460 durch die Zylinderwand 621 verschlossen ist. Somit wird ein Rückfördern der Monomerflüssigkeit 425 aus dem Fördervolumen 630 in den dritten Innenraum 420 unterbunden. Ein fortgeführtes Verschieben des Zylinders 620 in Richtung des ersten Behältnisses 200 führt zu einem Fördern der Monomerflüssigkeit 425 durch das erste Leitungsmittel 670 in den ersten Innenraum 220.

Die Vorrichtung 100 weist das Antriebselement 700 auf. Das Antriebselement 700 ist mehrteilig aufgebaut. Das Antriebselement 700 dient dazu, Kolben 610 und Zylinder 620 des Kolben-Zylinder-Systems 600 relativ zueinander axial zu bewegen. Ein relatives aufeinander zubewegen von Kolben 610 und Zylinder 620 führt, bei mit Monomerflüssigkeit 425 befülltem Fördervolumen 630, zu einem Verbringen der Monomerflüssigkeit 425 in den ersten Innenraum 220. Hierzu weist das Antriebselement 700 eine Getriebeeinheit 710 auf. Die Getriebeeinheit 710 weist ein erstes Vortriebsmittel 720 und ein zweites Vortriebsmittel 730 auf, welche durch ein Zusammenwirken eine Relativbewegung von Kolben 610 und Zylinder 620 zueinander ermöglichen. Das erste Vortriebsmittel 720 und das zweite Vortriebsmittel 730 wirken derart zusammen, dass eine Bewegung des einen Vortriebsmittel eine gerichtete Bewegung des anderen Vortriebsmittel auslöst. Beispielsweise kann eine Drehbewegung des einen Vortriebsmittels eine lineare Bewegung des anderen Vortriebsmittels auslösen. Das erste Vortriebsmittel 720 und das zweite Vortriebsmittel 730 können auf unterschiedliche Arten zusammenwirken, um eine Relativbewegung von Kolben 610 und Zylinder 620 auszulösen. In der gezeigten Ausgestaltungsform ist das erste Vortriebselement 720 zahnstangenartig und das zweite Vortriebselement 730 zumindest teilweise ritzelartig ausgestaltet. Das erste Vortriebsmittel 720 und das zweite Vortriebsmittel 730 wirken derart zusammen, dass bei einer rotatorischen Bewegung des zweiten Vortriebselement 730 um die Achse 750 gegen den Uhrzeigersinn der ritzelartig ausgestaltete Teil des zweiten Vortriebsmittels 730 in die zahnstangenartige Struktur des ersten Vortriebsmittels 720 greift und das erste Vortriebmittel 720 in Richtung des ersten Behältnisses 200 verschiebt. Damit das erste Vortriebsmittel 720 die durch das zweite Vortriebsmittel 730 ausgelöste Bewegung auf das Kolben-Zylinder-System 600 übertragen kann, sind in der gezeigten Ausgestaltungsform das erste Vortriebsmittel 720 und der Zylinder 620 einstückig ausgestaltet. In weiteren, nicht gezeigten, Ausgestaltungsformen kann das erste Vortriebsmittel 720 form- und oder kraftschlüssig mit dem Zylinder 620 oder form- und/oder kraftschlüssig mit dem Kolben 610 zusammenwirken oder das erste Vortriebsmittel 720 und der Kolben 610 sind einstückig ausgestaltet.

Die Drehbewegung des zweiten Vortriebselement 730 wird durch den Anwender der Vorrichtung 100 ausgelöst. Dazu ist das Antriebselement 700, wie bereits oben beschrieben, hebelartig ausgestaltet.

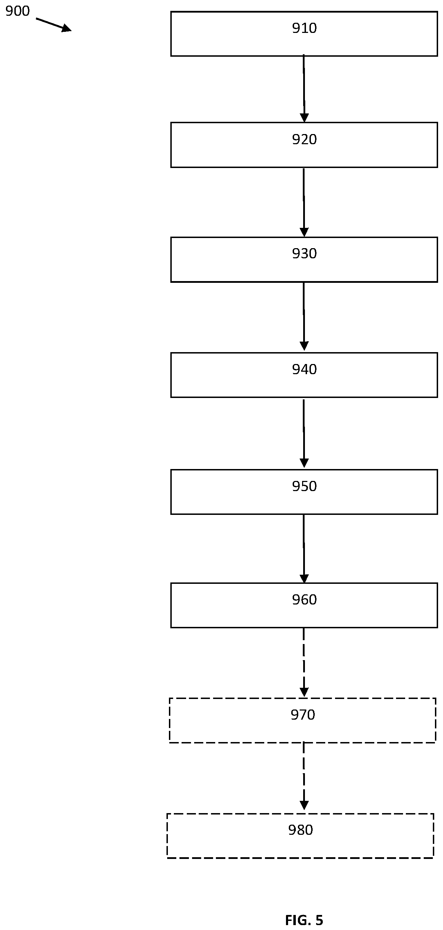

**Figur 5** zeigt ein Flussdiagramm enthalten die Schritte 910 bis 960, sowie die optionalen Schritte 970 und 980, eines Verfahrens 900 zur Bereitstellung eines Knochenzements aus zwei Ausgangskomponenten mittels der Vorrichtung 100. In einem ersten Schritt 910 wird das Kolben-Zylinder-System 600 in die erste Position überführt oder das Kolben-Zylinder-System 600 befindet sich bereits in der ersten Position. In der ersten Position ist das zweite Leitungsmittel 460 fluidleitend mit dem Fördervolumen 630 verbunden. Somit kann, eventuell nach dem Öffnen des Gefäßes 410, die Monomerflüssigkeit 425 in Schritt 920 in das Fördervolumen 630 fließen. Um ein Rückfließen der Monomerflüssigkeit 425 aus dem Fördervolumen 630 in den dritten Innenraum 420 zu verhindern, wird in einem Schritt 930 das Kolben-Zylinder-System 600 aus der ersten Position in die zweite Position überführt. Dadurch wird in Schritt 940 die fluidleitende Verbindung zwischen dem zweiten Leitungsmittel 460 und dem Fördervolumen 630 unterbunden. Dies geschieht vorzugsweise durch ein Verschieben des Zylinders 620 relativ zum Kolben 610 mittels des Antriebselements 700. In Schritt 950, wird die Monomerflüssigkeit 425 aus dem Fördervolumen 630 in den ersten Innenraum 220 gefördert. Dies geschieht vorzugsweise durch eine fortgeführte Verschiebung des Zylinders 620 gegen den Kolben 610. In Schritt 960 kommt es zur Ausbildung des Knochenzements aus Knochenzementpulvers 225 und Monomerflüssigkeit 425. Vorzugsweise erfolgt die Ausbildung des Knochenzements ohne mechanische Einwirkung durch eine Mischeinrichtung. Nach erfolgter Ausbildung des Knochenzements kann das erste Behältnis 200 von dem zweiten Behältnis 300 in Schritt 970 abgetrennt werden, beispielsweise durch das Lösen des Bajonettverschlusses 500. Anschließend kann der Knochenzement in Schritt 980 aus der Austragsvorrichtung 500 des ersten Behältnisses 200, eventuell nach vorherigem Anbringen eines Applizierschnorchels, an gewünschter Stelle appliziert werden.

Die in den Ansprüchen, der Beschreibung und in den Figuren offenbarten Merkmale können für verschiedene Ausführungsformen der beanspruchten Erfindung sowohl getrennt als auch in beliebiger Kombination miteinander wesentlich sein. Die für die Vorrichtung offenbarten Merkmale sind auch für das Verfahren offenbart und die für das Verfahren offenbarten Merkmale sind auch für die Vorrichtung offenbart.

### Bezugszeichen

- 100: Vorrichtung
- 200: erstes Behältnis
- 201: Behältniswand des ersten Behältnisses
- 210: erstes Ende des ersten Behältnisses
- 215: zweites Ende des ersten Behältnisses
- 220: erster Innenraum
- 225: Knochenzementpulver
- 260: erstes Anschlussmittel
- 300: zweites Behältnis
- 301: Behältniswand des zweiten Behältnisses
- 310: erstes Ende des zweiten Behältnisses
- 311: zweites Ende des zweiten Behältnisses
- 315: Flanschring
- 316: Hohlraum
- 320: zweiter Innenraum
- 350: Außenfläche
- 360: zweites Anschlussmittel
- 370: Standelement
- 400: Lagerelement
- 401: erstes Ende des Lagerelements
- 402: zweites Ende des Lagerelements
- 410: Gefäß
- 411: Glasampullenkopf
- 412: Glasampullenkörper
- 413: Glasampullenhals
- 415: Öffnungsvorrichtung
- 420: dritter Innenraum
- 425: Monomerflüssigkeit
- 460: zweites Leitungsmittel
- 500: Austragsvorrichtung
- 501: Außengewinde der Austragsvorrichtung
- 510: Verschluss
- 511: Verschlussstift
- 512: Verschlussinnengewinde
- 515: Drehflügel des Verschlusses
- 550: Bajonettverschluss
- 600: Kolben-Zylinder-System
- 610: Kolben
- 620: Zylinder
- 621: Zylinderwand
- 625: Zylinderdurchführung
- 630: Fördervolumen
- 670: erstes Leitungsmittel
- 700: Antriebselement
- 701: erster Teil des Antriebselements
- 702: zweiter Teil des Antriebselements
- 710: Getriebeeinheit
- 720: erste Vortriebsmittel
- 730: zweite Vortriebsmittel
- 750: Achse
- 800: Austragskolben
- 801: Außenfläche des Austragskolbens
- 802: Dichtungsringe
- 805: Durchführung des Austragskolbens
- 804: Dichtungselement
- 900: Verfahren zur Bereitstellung eines Knochenzements aus zwei Ausgangskomponenten
- 910: Überführen des Kolbenzylindersystems in die erste Position
- 920: Fördern der Monomerflüssigkeit
- 930: Überführen des Kolbenzylinder in die zweite Position
- 940: Verschließen der Verbindung
- 950: Fördern der Monomerflüssigkeit
- 960: Ausbildung des Knochenzements
- 970: Trennen von erstem Behältnis und zweitem Behältnis
- 980: Applizieren des Knochenzements

## Patentansprüche

1. Vorrichtung (100) zum Bereitstellen eines Knochenzements aus zwei Ausgangskomponenten, umfassend
ein zylinderartiges erstes Behältnis (200) mit einem ersten Innenraum (220), in dem ein Knochenzementpulver (225) als erste Ausgangskomponente lagerbar ist,
ein zylinderartiges zweites Behältnis (300) mit einem zweiten Innenraum (320), in dem ein Kolben-Zylinder-System (600) angeordnet ist, aufweisend einen in einem Zylinder (620) angeordneten Kolben (610), wobei Kolben (610) und Zylinder (620) relativ zueinander axial bewegbar sind und das Kolben-Zylinder-System (600) ein Fördervolumen (630) aufweist,
ein erstes Leitungsmittel (670) welches das Fördervolumen (630) fluidleitend mit dem ersten Innenraum (220) verbindet,
ein Lagerelement (400) mit einem dritten Innenraum (420), in dem eine Monomerflüssigkeit (425) als zweite Ausgangskomponente lagerbar ist,
wobei das erste Behältnis (200) und das zweite Behältnis (300) axial miteinander verbunden sind,
**dadurch gekennzeichnet, dass**
das Lagerelement (400) an einer Außenfläche (350) des zweiten Behältnisses (300) angeordnet ist,
ein fluidleitendes zweites Leitungsmittel (460) das Fördervolumen (630) und den dritten Innenraum (420) verbindet,
das Kolben-Zylinder-System (600)
• in einer ersten Position eine fluidleitende Verbindung zwischen dem zweiten Leitungsmittel (460) und dem Fördervolumen (630) eröffnet und
• in einer zweiten Position die fluidleitende Verbindung zwischen dem zweiten Leitungsmittel (460) und dem Fördervolumen (630) verschließt, und
die Vorrichtung (100) ein Antriebselement (700) umfasst, wobei das Antriebselement (700) über eine Getriebeeinheit (710) auf das Kolben-Zylinder-System (600) einwirkt.

2. Vorrichtung (100) nach Anspruch 1, **dadurch gekennzeichnet, dass** die Getriebeeinheit (710) ein zahnstangenartig ausgebildetes erstes Vortriebsmittel (720) und ein wenigstens partiell ritzelartig ausgestaltetes zweites Vortriebsmittel (730) umfasst.

3. Vorrichtung (100) nach Anspruch 1 oder 2, **dadurch gekennzeichnet, dass** das Antriebselement (700) derart mit dem Zylinder (620) verbunden ist, dass eine rotatorische Bewegung des Antriebselements (700) in einer linearen Bewegung des Zylinders (620) resultiert.

4. Vorrichtung (100) nach einem der vorhergehenden Ansprüche 1 bis 3, **dadurch gekennzeichnet, dass** das Antriebselement (700) hebelartig ausgestaltet und an der Außenfläche (350) des zweiten Behältnisses (300) angeordnet ist, insbesondere, dass das Antriebselement (700) um eine Achse (750) drehbar gelagert ist, bevorzugt um wenigstens 90 Grad, insbesondere wenigstens 60 Grad drehbar gelagert ist, insbesondere dass die Achse (750) im Wesentlichen rechtwinklig zu einer Längsachse der Vorrichtung (100) angeordnet ist.

5. Vorrichtung (100) nach einem der vorhergehenden Ansprüche 1 bis 4, **dadurch gekennzeichnet, dass** der Zylinder (620) axial in dem zweiten Innenraum (320) bewegbar ist.

6. Vorrichtung (100) nach einem der vorhergehenden Ansprüche 1 bis 5, **dadurch gekennzeichnet, dass** der Kolben (610) form- und/oder kraftschlüssig mit dem zweiten Behältnis (100) verbunden ist.

7. Vorrichtung (100) nach einem der vorhergehenden Ansprüche 4 bis 6, **dadurch gekennzeichnet, dass** das Antriebselement (700) derart mit dem Zylinder (620) verbunden ist, dass eine rotatorische Bewegung des Antriebselements (700) um die Achse (750) in einer linearen Bewegung des Zylinders (620) resultiert.

8. Vorrichtung (100) nach einem der vorhergehenden Ansprüche 1 bis 4, **dadurch gekennzeichnet, dass** der Kolben (610) axial in dem zweiten Innenraum (320) bewegbar ist.

9. Vorrichtung (100) nach einem der vorhergehenden Ansprüche 1 bis 4 oder 8, **dadurch gekennzeichnet, dass** der Zylinder (620) form- und/oder kraftschlüssig mit dem zweiten Behältnis (300) verbunden ist.

10. Vorrichtung (100) nach einem der vorhergehenden Ansprüche 4, oder 8, **dadurch gekennzeichnet, dass** das Antriebselement (700) derart mit dem Kolben (610) verbunden ist, dass eine rotatorische Bewegung des Antriebselements (700) um die Achse (750) in einer linearen Bewegung des Kolbens (610) resultiert.

11. Vorrichtung (100) nach einem der vorangehenden Ansprüche, **dadurch gekennzeichnet, dass** die Vorrichtung (100), insbesondere das erste Behältnis (200), mischeinrichtungsfrei ist.

12. Vorrichtung (100) nach einem der vorhergehenden Ansprüche, **dadurch gekennzeichnet, dass** das Lagerelement (400) ein Gefäß (410) mit der Monomerflüssigkeit (425) beinhaltet.

13. Vorrichtung (100) nach Anspruch 12, **dadurch gekennzeichnet, dass** das Lagerelement (400) eine Öffnungsvorrichtung (415) für das Gefäß (410) aufweist, insbesondere dass die Öffnungsvorrichtung (415) ein Anstechdorn oder eine Bruchkante ist.

14. Vorrichtung (100) nach Anspruch 12 oder 13, **dadurch gekennzeichnet, dass** das Gefäß (410) eine Glasampulle oder ein Beutel ist.

15. Vorrichtung (100) nach einem der vorhergehenden Ansprüche, **dadurch gekennzeichnet, dass** in dem ersten Behältnis (200) ein axial verschiebbarer Austragskolben (800) angeordnet ist.

16. Vorrichtung (100) nach einem der vorhergehenden Ansprüche, **dadurch gekennzeichnet, dass** das Knochenzementpulver (225) in dem ersten Behältnis (200) zwischen dem Austragskolben (800) und einer Austragsvorrichtung (500) angeordnet ist.

17. Vorrichtung (100) nach einem der vorhergehenden Ansprüche, **dadurch gekennzeichnet, dass** das erste Behältnis (200) ein erstes Anschlussmittel (260) und das zweite Behältnis (300) ein zweites Anschlussmittel (360) aufweisen, um das erste Behältnis (200) und das zweite Behältnis (300) zu verbinden, insbesondere form- und/oder kraftschlüssig zu verbinden.

18. Vorrichtung (100) nach dem vorhergehenden Anspruch 17, **dadurch gekennzeichnet, dass** das erste Anschlussmittel (260) und das zweite Anschlussmittel (360) einen Bajonettverschluss bilden.

19. Vorrichtung (100) nach dem vorhergehenden Anspruch 18, **dadurch gekennzeichnet, dass** das erste Anschlussmittel (260) als ein Außengewinde und das zweite Anschlussmittel (360) als ein Innengewinde ausgestaltet sind, wobei das Außengewinde und das Innengewinde form- und/oder kraftschlüssig miteinander verbindbar sind.

20. Verfahren zur Bereitstellung eines Knochenzements aus zwei Ausgangkomponenten mittels einer Vorrichtung (100) gemäß einem der vorhergehenden Ansprüche, zumindest umfassend die folgenden sequenziellen Teilschritte:
a) Überführen des Kolben-Zylinder-Systems (600) in die erste Position,
b) Fördern der Monomerflüssigkeit (425) mittels der fluidleitenden Verbindung in das Fördervolumen (630),
c) Überführen des Kolben-Zylinder-Systems (600) aus der ersten Position in die zweite Position,
d) Verschließen der fluidleitenden Verbindung zwischen dem zweiten Leitungsmittel (460) und dem Fördervolumen (630),
e) Fördern der Monomerflüssigkeit (425) in den ersten Innenraum (220),
f) Ausbildung des Knochenzements aus Knochenzementpulver (225) und Monomerflüssigkeit (425).
